# EUROPEAN PATENT APPLICATION

(11) **EP 4 592 394 A1**
(43) Date of publication of application: **30.07.2025**
(21) Application number: 23868590.3
(22) Date of filing: 20.09.2023
(51) Int. Cl.: C12N 15/82, C12N 15/10, C12N 9/78, C07K 14/415

(54) **BASE EDITING OF PLANT CELL ORGANELLE DNA**

(30) Priority: 21.09.2022 KR 20220119363
(71) Applicant: Institute for Basic Science, Yuseong-gu Daejeon 34126 (KR)
(72) Inventor: KIM, Jin-Soo, Seoul 08831 (KR); HONG, Sunghyun, Daegu 42779 (KR); MOK, Young Geun, Seoul 08833 (KR); BAE, Su-Ji, Daejeon 34046 (KR)
(74) Representative: RGTH
(86) International application number: PCT/KR2023/014300
(87) International publication number: WO 2024/063538

(57) **Abstract**

The present invention relates to a composition for base editing of plant cell organelle DNA and, in particular, to a composition and method for editing adenine to guanine and cytosine to thymine in plant cell organelle DNA.

## Description

### [Technical Field]

The present invention relates to a composition for base editing of plant cell organelle DNA, and particularly to a composition and editing method for simultaneously editing adenine to guanine and cytosine to thymine in plant cell organelle DNA.

### [Background Art]

A fusion protein with a DNA binding protein and a deaminase linked enables single-nucleotide conversion in a targeted manner to perform targeted nucleotide substitution or base editing in the genome without generating DNA double-strand breaks (DSBs), to edit point mutations causing genetic disorders, or to introduce desired single-nucleotide variation in prokaryotes, humans, and other eukaryotes.

Programmable genome editing tools such as ZFN (zinc finger nuclease), TALEN (transcription activator-like effector nuclease), CRISPR (clustered regularly interspaced short palindromic repeat) systems and CRISPR-associated protein 9 (Cas9) variants without nucleolytic efficiency, and base editing technology of nucleotide deaminase proteins have been developed for genetic research of plants and improvement of crop traits through changes in base sequences. However, these tools are not suitable for editing DNA sequences in plant organelles, including mitochondria and chloroplasts, mainly because of difficulties in delivering guide RNAs to organelles or simultaneous expression of two components in organelles. Plant cell organelles encode many essential genes required for photosynthesis and respiration. Methods or tools for editing organelle genes are very necessary for studying the function of the genes or improving crop productivity and traits. For example, targeted mutations in the mitochondrial atp6 gene may lead to male sterility, a trait useful in reproduction, and specific point mutations in the 16S rRNA gene of the chloroplast genome may lead to antibiotic resistance.

Bacterial toxin DddAtox is an enzymatic moiety in bacterial toxins derived from Burkholderia cenocepacia and is responsible for cytosine deamination within double-stranded DNA. As an example of a deaminase, DddAtox is toxic in cells, and thus, to avoid toxicity in host cells, DddAtox is split into two inactive splits, each of which is linked to a DNA binding protein designed to bind to DNA to make a functional DdCBE pair.

Under this technical background, it has been confirmed that both A-to-G base editing and C-to-T base editing may occur in plant cell organelle DNA, arriving at the present invention.

### [Summary of the Invention]

It is an object of the present invention to provide a composition for base editing of plant cell organelle DNA, which includes a DNA binding protein, a cytosine deaminase or a cytosine deaminase in split form, and an adenine deaminase or a nucleic acid encoding the same.

It is another object of the present invention to provide a method for editing plant cell organelle DNA.

To achieve the above object, the present invention provides a composition for base editing of plant cell organelle DNA, comprising a DNA binding protein or a nucleic acid encoding the same; cytosine deaminase or a first and second splits derived from cytosine deaminase or a nucleic acid encoding the same; and adenine deaminase or a nucleic acid encoding the same.

The present invention also provides a method for editing a base of plant cell organelle DNA, including treating a plant cell with the composition.

### [Description of Drawings]

FIG. 1 is a schematic diagram of DNA cloning for producing transgenic plants using Agrobacterium.
FIG. 2 shows a transgenic plant targeting the psaA gene, showing: (a) base editing efficiency and positions in each plant; (b) the plant phenotype; and (c) the results of amino acid sequence changes caused by DNA base editing.
FIG. 3 is a transgenic plant targeting the rbcL gene, showing: (a) base editing efficiency and positions in each plant; and (b) the results of amino acid sequence changes caused by DNA base editing.
FIG. 4 shows the results of confirming the base editing efficiency and positions in transgenic plants targeting the rrn16S gene.

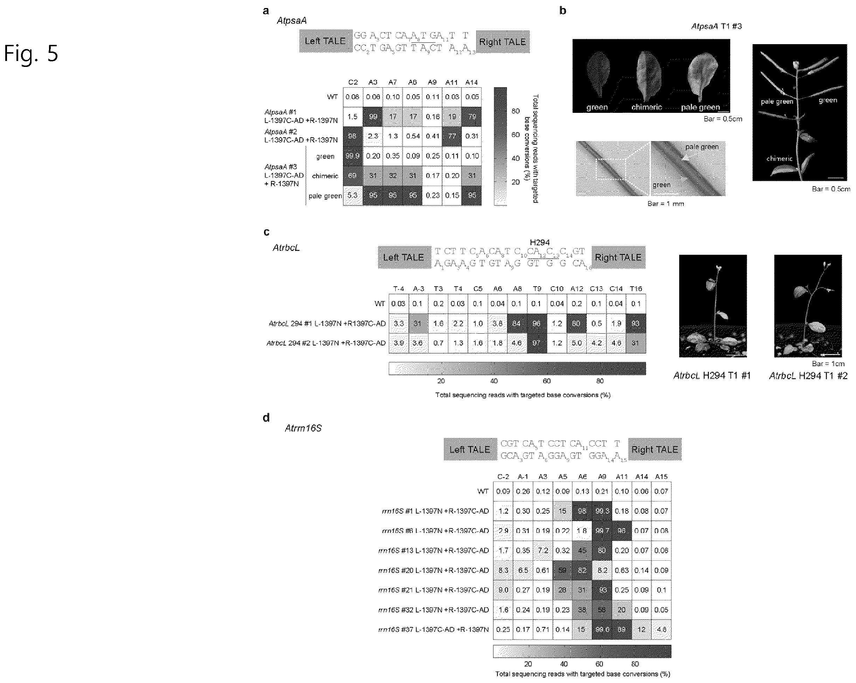

FIG. 5 illustrates the base editing efficiency and phenotype of first-generation transgenic plants targeting the psaA, rbcL, and rrn16S genes in the chloroplasts of Arabidopsis thaliana, showing: (a) the base editing efficiency and positions of psaA #1, #2, and #3, where not only base editing of adenine but also base editing of cytosine occur simultaneously; (b) the phenotype of psaA #3, exhibiting green, chimeric, and pale green traits; (c) the base editing efficiency, positions, and phenotype of rbcL #1 and #2; and (d) the base editing efficiency of first-generation transgenic plants targeting the rrn16S gene.
FIG. 6 illustrates the base editing efficiency and phenotype of second-generation transgenic plants, showing: (a) the phenotype of second-generation psaA #3 transgenic plants, (b) base editing efficiency and positions in psaA #3; (c) the phenotype of second-generation rrn16S transgenic plants exhibiting spectinomycin resistance, (d) base editing efficiency and positions in rrn16S transgenic plants; and (e) PCR analysis confirming the presence or absence of foreign genes in transgenic plants, which confirmed that rrn16S #9-1 and #9-3 lack foreign genes.
FIG. 7 shows the results of confirming the base editing positions and efficiency of spectinomycin-resistant and spectinomycin-sensitive plants among the second-generation rrn16S transgenic plants.
FIG. 8 shows off-target base mutations identified through whole chloroplast genome analysis of psaA #1 (a), psaA #3 chimeric (b), psaA #3 pale green (c), rrn16S #1 (d), rrn16S #6 (e), and wild-type Col-0 (f), revealing that no distinct off-target base mutations were observed in transgenic plants compared to the wild type
FIG. 9 shows the efficiency of base editing of the lettuce mitochondrial gene atp6.

### [Detailed Description of the Invention and Preferred Embodiments]

Unless otherwise defined, all technical and scientific terms used herein have the same meanings as typically understood by those skilled in the art to which the present invention belongs. In general, the nomenclature used herein is well known in the art and is typical.

The only method of base editing of plant cell organelle DNA was to edit cytosine to thymine by attaching DddAtox to TALE. The technical task according to the present invention is intended to edit the base of cell organelle DNA by attaching DddAtox and an adenine deaminase to TALE. By base-editing the cell organelle DNA, which was conventionally impossible, it is possible to develop functional plants, such as plants with herbicide resistance.

The inventors of the present application manufactured a base editor capable of performing both A-to-G and C-to-T base editing by linking a DddAtox cytosine deaminase and an adenine deaminase to a TALE or ZFP protein capable of binding to DNA.

Based on the above, from one aspect, the present invention relates to a composition for editing organellar DNA in plant cells, comprising: DNA binding protein or nucleic acid encoding said DNA binding protein; cytosine deaminase, or first and second splits derived from cytosine deaminase, or nucleic acid encoding the same; and adenine deaminase or nucleic acid encoding said adenine deaminase, wherein each of said first and second splits independently binds to DNA binding protein, and the composition simultaneously performs adenine-to-guanine editing and cytosine-to-thymine editing in plant organellar DNA.

As used herein, the term "editing" may be used interchangeably with "proofreading" and refers to a method of altering a nucleic acid sequence by selective deletion of a specific genomic target. The specific genomic target includes, but is not limited to, a chromosomal region, a gene, a promoter, an open reading frame, or any nucleic acid sequence.

As used herein, the "single base" refers to one, and only one, nucleotide within a nucleic acid sequence. In the context of single-base editing, this means that the base at a specific position within the nucleic acid sequence is replaced with a different base. This replacement may occur by many mechanisms, including but not limited to, substitution or modification.

As used herein, the term "target" or "target site" refers to a pre-identified nucleic acid sequence of any composition and/or length. The target site includes, but is not limited to, a chromosomal region, a gene, a promoter, an open reading frame, or any nucleic acid sequence.

As used herein, the term "on-target" refers to a subsequence of a specific genomic target that may be completely complementary to a programmable DNA binding domain and/or a single guide RNA sequence.

As used herein, the term "off-target" refers to a subsequence of a specific genomic target that may be partially complementary to a programmable DNA binding domain and/or a single guide RNA sequence.

The present invention includes a first split part and a second split part derived from a cytosine deaminase or variants thereof, wherein the cytosine deaminase binds to a DNA binding protein or each of the first and second splits independently binds to the DNA binding protein.

The cytosine deaminase is an enzyme that removes an amino group, and enables cytosine (C) to uridine (U) conversion.

The cytosine deaminase may be used. For example, there are types of cytosine deaminase, such as APOBEC1 (apolipoprotein B editing complex 1) and AID (activation-induced deaminase), but most DNA deaminases only work on single-stranded DNA and may not be suitable for base editing by linkage to a DNA binding protein. Specifically, the cytosine deaminase may be derived from a deaminase (DddA) acting on double-stranded DNA or an orthologue thereof. More specifically, the cytosine deaminase may be a double-stranded DNA-specific bacterial cytosine deaminase.

The cytosine deaminase is provided in split form, the cytosine deaminase includes a first split and a second split, and each of the first split and the second split has no deaminase activity.

The cytosine deaminase may include the sequence of SEQ ID NO: 1 corresponding to the tox split in a full-length cytosine deaminase. The cytosine deaminase includes a first split and a second split, and each of the first split and the second split has no deaminase activity.

(SEQ ID NO: 1)
GSYALGPYQISAPQLPAYNGQTVGTFYYVNDAGGLESKVFSSGGPTPYPNYANAGHVEGQSALF
MRDNGISEGLVFHNNPEGTCGFCVNMTETLLPENAKMTVVPPEGAIPVKRGATGETKVFTGNNSNSPKS
PTKGGC

In one embodiment, the first split or the second split of the cytosine deaminase may include a sequence from the N-terminus to at least one selected from the group consisting of G33, G44, A54, N68, G82, N98, G108 in the sequence of SEQ ID NO: 1. The first split or the second split of the cytosine deaminase may include a sequence from at least one selected from the group consisting of G34, P45, G55, N69, T83, A99, and A109 to the C-terminus in the sequence of SEQ ID NO: 1.

Specifically, the cytosine deaminase may include a first split of SEQ ID NO: 2 (G1333-N) and a second split of SEQ ID NO: 3 (G1333-C), a first split of SEQ ID NO: 4 (G1397-N) and a second split of SEQ ID NO: 5 (G1397-C), a first split of SEQ ID NO: 2 (G1333-N) and a second split of SEQ ID NO: 5 (G1397-C), or may include a first split of SEQ ID NO: 4 (G1397-N) and a second split of SEQ ID NO: 2 (G1333-C).

Unless otherwise defined, all technical and scientific terms used herein have the same meanings as typically understood by those skilled in the art to which the present invention belongs. In general, the nomenclature used herein is well known in the art and is typical.

The only method of base editing of plant cell organelle DNA was to edit cytosine to thymine by attaching DddAtox to TALE. The technical task according to the present invention is intended to edit the base of cell organelle DNA by attaching DddAtox and an adenine deaminase to TALE. By base-editing the cell organelle DNA, which was conventionally impossible, it is possible to develop functional plants, such as plants with herbicide resistance.

The inventors of the present application manufactured a base editor capable of performing both A-to-G and C-to-T base editing by linking a DddAtox cytosine deaminase and an adenine deaminase to a TALE or ZFP protein capable of binding to DNA.

Based on the above, from one aspect, the present invention relates to a composition for editing organellar DNA in plant cells, comprising: DNA binding protein or nucleic acid encoding said DNA binding protein; cytosine deaminase, or first and second splits derived from cytosine deaminase, or nucleic acid encoding the same; and adenine deaminase or nucleic acid encoding said adenine deaminase, wherein each of said first and second splits independently binds to DNA binding protein, and the composition simultaneously performs adenine-to-guanine editing and cytosine-to-thymine editing in plant organellar DNA.

As used herein, the term "editing" may be used interchangeably with "proofreading" and refers to a method of altering a nucleic acid sequence by selective deletion of a specific genomic target. The specific genomic target includes, but is not limited to, a chromosomal region, a gene, a promoter, an open reading frame, or any nucleic acid sequence.

As used herein, the "single base" refers to one, and only one, nucleotide within a nucleic acid sequence. In the context of single-base editing, this means that the base at a specific position within the nucleic acid sequence is replaced with a different base. This replacement may occur by many mechanisms, including but not limited to, substitution or modification.

As used herein, the term "target" or "target site" refers to a pre-identified nucleic acid sequence of any composition and/or length. The target site includes, but is not limited to, a chromosomal region, a gene, a promoter, an open reading frame, or any nucleic acid sequence.

As used herein, the term "on-target" refers to a subsequence of a specific genomic target that may be completely complementary to a programmable DNA binding domain and/or a single guide RNA sequence.

As used herein, the term "off-target" refers to a subsequence of a specific genomic target that may be partially complementary to a programmable DNA binding domain and/or a single guide RNA sequence.

The present invention includes a first split part and a second split part derived from a cytosine deaminase or variants thereof, wherein the cytosine deaminase binds to a DNA binding protein or each of the first and second splits independently binds to the DNA binding protein.

The cytosine deaminase is an enzyme that removes an amino group, and enables cytosine (C) to uridine (U) conversion.

The cytosine deaminase may be used. For example, there are types of cytosine deaminase, such as APOBEC1 (apolipoprotein B editing complex 1) and AID (activation-induced deaminase), but most DNA deaminases only work on single-stranded DNA and may not be suitable for base editing by linkage to a DNA binding protein. Specifically, the cytosine deaminase may be derived from a deaminase (DddA) acting on double-stranded DNA or an orthologue thereof. More specifically, the cytosine deaminase may be a double-stranded DNA-specific bacterial cytosine deaminase.

The cytosine deaminase is provided in split form, the cytosine deaminase includes a first split and a second split, and each of the first split and the second split has no deaminase activity.

The cytosine deaminase may include the sequence of SEQ ID NO: 1 corresponding to the tox split in a full-length cytosine deaminase. The cytosine deaminase includes a first split and a second split, and each of the first split and the second split has no deaminase activity.
(SEQ ID NO: 2) Wild-type DddAtox G1333-N
   GSYALGPYQISAPQLPAYNGQTVGTFYYVNDAGGLESKVFSSGG
(SEQ ID NO: 33) 
GGCTCTGGTTCCTACGCCCTGGGTCCATATCAGATTAGTGCTCCCCAACTCCCCGCCTACAAC
GGTCAGACAGTGGGGACCTTTTACTATGTCAACGACGCCGGGGGATTGGAATCCAAGGTTTTCTCTAG
CGGTGGG
(SEQ ID NO: 3) Wild-type G1333-C 
PTPYPNYANAGHVEGQSALFMRDNGISEGLVFHNNPEGTCGFCVNMTETLLPENAKMTVVPP
EGAIPVKRGATGETKVFTGNSNSPKSPTKGGC
(SEQ ID NO: 34) 
CCAACACCTTATCCTAACTACGCTAACGCCGGGCACGTCGAGGGGCAGTCAGCTCTTTTTATG
AGAGATAACGGCATTAGCGAAGGGCTTGTGTTCCATAATAATCCTGAGGGCACCTGTGGCTTCTGTGTA
AATATGACCGAAACACTTCTGCCTGAGAACGCTAAAATGACTGTCGTACCACCCGAAGGCGCAATCCCA
GTTAAACGGGGCGCAACCGGCGAAACCAAAGTATTCACCGGAAACAGCAATAGTCCAAAGTCCCCCA
CCAAGGGAGGTTGC
(SEQ ID NO: 4) Wild-type DddAtox G1397-N 
GSYALGPYQISAPQLPAYNGQTVGTFYYVNDAGGLESKVFSSGGPTPYPNYANAGHVEGQSALF
MRDNGISEGLVFHNNPEGTCGFCVNMTETLLPENAKMTVVPPEG
(SEQ ID NO: 35) 
GGTAGCTACGCACTTGGTCCTTACCAGATTAGCGCACCCCAACTCCCCGCCTATAATGGTCAA
ACCGTCGGGACCTTTTACTACGTAAACGATGCTGGTGGGCTGGAATCCAAAGTATTCTCCTCAGGGGG
CCCTACACCCTACCCCAACTACGCCAATGCTGGTCATGTAGAAGGGCAGTCAGCACTGTTTATGCGCGA
TAATGGTATAAGCGAGGGGTTGGTCTTCCATAACAACCCAGAGGGTACTTGTGGCTTCTGTGTGAATAT
GACTGAAACCCTTCTGCCCGAAAATGCCAAGATGACTGTCGTCCCACCTGAAGGC
(SEQ ID NO: 5) Wild-type DddAtox G1397-C
   AIPVKRGATGETKVFTGNNSNSPKSPTKGGC
(SEQ ID NO: 36)

GCCATACCTGTGAAGCGGGGAGCAACAGGGGAGACAAAGGTGTTCACAGGCAACTCTAAC
AGTCCAAAGAGCCCCACCAAAGGCGGGTGT

The above G1333N, G1333C, G1397N, and G1397C in combination may be used as a deaminase in split form. Specifically, there may be provided in the form of Left-G1333-N + Right-G1333-C, Left-G1397-N + Right-G1397-C, Left-G1397-N + Right-G1333-C, or Left-G1333-N + Right-G1397-C.

The DNA binding protein may be, for example, a zinc finger protein, a TALE (transcription activator-like effector) array, or a combination thereof.

The zinc finger motif of the zinc finger protein has a DNA binding domain, and the C-terminus of the finger specifically recognizes a DNA sequence. DNA binding proteins including 3-6 zinc finger motifs recognize DNA sequences.

In one embodiment, each of the first split of the cytosine deaminase and the second split of the cytosine deaminase may bind to the N-terminus or C-terminus of the zinc finger protein.

Binding of the C-terminus of the zinc finger protein (ZF-Left) to the N-terminus of the first split of the cytosine deaminase, and binding of the C-terminus of the zinc finger protein (ZF-Right) to the N-terminus of the second split of the cytosine deaminase (CC configuration);
Binding of the N-terminus of the zinc finger protein (ZF-Left) to the C-terminus of the first split of the cytosine deaminase, and binding of the C-terminus of the zinc finger protein (ZF-Right) to the N-terminus of the second split of the cytosine deaminase (NC configuration);
Binding of the C-terminus of the zinc finger protein (ZF-Left) to the N-terminus of the first split of the cytosine deaminase, and binding of the N-terminus of the zinc finger protein (ZF-Right) to the C-terminus of the second split of the cytosine deaminase (CN configuration); or
Binding of the N-terminus of the zinc finger protein (ZF-Left) to the C-terminus of the first split of the cytosine deaminase, and binding of the N-terminus of the zinc finger protein (ZF-Right) to the C-terminus of the second split of the cytosine deaminase (NN configuration).

The ZF-Left may comprise the sequence of the following SEQ ID NO: 6:

[SEQ ID NO: 6]

GIHGVPAAMAERPFQCRICMRNFSDRSNLSRHIRTHTGEKPFACDICGRKFAISSNLNSHTKIHT
GSQKPFQCRICMRNFSRSDNLARHIRTHTGEKPFACDICGRKFATSGNLTRHTKIHLR.

The ZF-Right may include the following SEQ ID NO: 7:

[SEQ ID NO: 7]
GIHGVPAAMAERPFQCRICMRNFSRSDNLSVHIRTHTGEKPFACDICGRKFAQKINLQVHTKIH
TGEKPFQCRICMRNFSRSDVLSEHIRTHTGEKPFACDICGRKFAQRNHRTTHTKIHLR.

The sequence of ZF may vary depending on the DNA target. ZF may be customized depending on the DNA target sequence. Since ZF recognizes 3 bp of DNA, it is possible to construct a ZF combination that recognizes 9-18 bp of DNA by connecting 3-6 ZFs. For example, the construction is possible using a library that includes modules such as GNN, TNN, CNN, or ANN.

In some cases, the zinc finger protein may be linked to the deaminase via a linker. The linker may be a peptide linker including 2 to 40 amino acid residues. The linker may be, for example, a linker having a length of 2aa, 5aa, 10aa, 16aa, 24aa, or 32aa, but is not limited thereto.

In one embodiment, the linker may include:
2a.a linker: GS;
5a.a linker: TGEKQ (SEQ ID NO: 8);
10a.a linker: SGAQGSTLDF (SEQ ID NO: 9);
16a.a linker: SGSETPGTSESATPES (SEQ ID NO: 10);
24 a linker: SGTPHEVGVYTLSGTPHEVGVYTL (SEQ ID NO: 45); or
32a.a linker: GSGGSSGGSSGSETPGTSESATPESSGGSSGGS (SEQ ID NO: 11).

In a specific embodiment according to the present invention, the split deaminase and the zinc finger protein may be linked via a linker, the zinc finger protein binds to the N-terminus of the half deaminase including the first split, and the zinc finger protein binds to the N-terminus of the half deaminase including the second split. As such, C-to-T base conversion may occur in the spacer between sites to which the left and right ZFPs are attached. Both the left and right ZFPs were confirmed to exhibit high editing efficiency when linked respectively to the half deaminase including the first split and the half deaminase including the second split via a 24 a.a linker.

The TAL effector (TALE) is composed of repeats, each 33-34 amino acids long, with about 9 domains repeated (repeated variant domains (RVDs)). The TAL effector may recognize one nucleotide per domain and may bind to a specific DNA sequence depending on the 12th-13th amino acid sequence (HD->cytosine, NI->adenine, NG->thymine, NN->guanine). The TAL effector (TALE) recognizes a single DNA strand within the target site. The distance between target sites may be 12-14 nucleotides.

The TALE domain indicates a protein domain that binds to a nucleotide in a sequence-specific manner by combination of one or more TALE-repeat units. The TALE domain includes at least one TALE-repeat unit, particularly 1 to 30 TALE-repeat units, but is not limited thereto. TALE-repeat is a site that recognizes a specific nucleotide sequence in the TALE domain.

The TALE domain includes a backbone structure that comprises a region including the N-terminus of TALE and a region including the C-terminus of TALE.

| Name | Sequence | No. |
|---|---|---|
| First TALE N-term | GATATAGCTGATTTGAGGACCCTCGGGTACTCACAGCAGCAACAAGAAAAAATCAAA CCAAAGGTACGTAGCACGGTTGCCCAACATCATGAAGCCCTAGTTGGTCACGGATTT ACTCATGCCCATATTGTTGCACTGAGTCAACACCCTGCTGCATTAGGCACAGTGGCTG TGAAATATCAAGATATGATTGCTGCTCTTCCT GAGGCCACACACGAGGCAATTGTGGGTGTTGGAAAACAATGGTCTGGTGCGCGAG CGCTTGAAGCTCTCCTAACCGTAGCTGGAGAGTTGAGAGGCCCACCGCTTCAGCTT GACACAGGGCAGCTGTTGAAGATAGCAAAGAGGGGGGGTGTAACTGCTGTTGAAGC AGTCCATGCATGGAGAAATGCATTAACTGGAGCTCCA | 37 |
| | DIADLRTLGYSQQQQEKIKPKVRSTVAQHHEALVGHGFTHAHIVALSQHPAALGTVAVK YQDMIAALPEATHEAIVGVGKQWSGARALEALLTVAGELRGPPLQLDTGQLLKIAKRG GVTAVEAVHAWRNALTGAP | 12 |
| Secon d TALE N-term | GATATTGCTGATCTTCGAACATTGGGTTATTCTCAGCAGCAACAAGAGAAAATCAAA CCGAAAGTCCGTTCAACCGTAGCTCAACATCATGAAGCCCTTGTTGGTCATGGATTTA CTCATGCTCACATTGTTGCTCTAAGTCAGCACCCTGCCGCGCTAGGCACAGTTGCCGT CAAGTATCAAGATATGATAGCTGCTTTACCTG AAGCAACCCACGAGGCAATAGTGGGGGTTGGGAAGAGGGGTGCAGGCGCCAGGG CACTGGAGGCACTCTTAACAGTAGCTGGAGAACTTCGGGGACCACCATTGCAGCTT GATACTGGACAACTCTTGAAGATCGCAAAAAGAGGGGGTGTGACTGCTGTGGAAGC TGTTCATGCATGGAGAAATGCTTTGACGGGAGCACCCCTG | 38 |
| | DIADLRTLGYSQQQQEKIKPKVRSTVAQHHEALVGHGFTHAHIVALSQHPAALGTVAVK YQDMIAALPEATHEAIVGVGKRGAGARALEALLTVAGELRGPPLQLDTGQLLKIAKRGG VTAVEAVHAWRNALTGAPL | 13 |
| First TALE C-term | AGCATAGTTGCTCAGCTAAGTAGACCTGATCCAGCACTTGCTGCATTGACGAACGAT CATCTCGTAGCGCTGGCTTGTTTGGGCGGCAGGCCAGCCTTGGACGCCGTGAAGAA AGGATTAGGT | 39 |
| | SIVAQLSRPPDPALAALTNDHLVALACLGGRPALDAVKKGLG | 14 |
| Secon d TALE C-term | TCGATAGTAGCACAATTAAGCAGGCCTGACCCTGCACTTGCTGCTCTTACAAATGATC ATCTTGTTGCTCTGGCCTGTTTGGGTGGGCGTCCCGCGTTGGATGCTGTTAAGAAAG GACTTGGT | 40 |
| | SIVAQLSRPPDPALAALTNDHLVALACLGGRPALDAVKKGLG | 15 |

Depending on the binding position of the TALE domain based on the cleavage site, a single TALE array or a first TALE array and a second TALE array may bind.

A first TALE (left TALE) may bind to the first split of the cytosine deaminase, and a second TALE (right TALE) may bind to the second split of the cytosine deaminase. The first TALE and the second TALE respectively have structures of N'-TALE-first split-C' and N'-TALE-second split-C'.

The TALE array may be customized depending on the target DNA sequence. The TALE array is configured such that modules composed of 33-35 amino acid residues are repeatedly arranged, and the TALEs are derived from the plant pathogen Xanthomonas and each module recognizes each of A, C, G, and T bases and binds to DNA. The base specificity of each module is determined by the 12th and 13th amino acid residues, which are called repeat variable di-residue (RVD). For example, a module where RVD is NN recognizes G, NI recognizes A, HD recognizes C, and NG recognizes T. The TALE array is composed of at least 14 to 18 modules and may be designed to recognize 15-20 bp of a target DNA sequence.

A mutated form of Cas protein may be included. This may refer to a mutation that eliminates endonuclease activity that cleaves double-stranded DNA, including, for example, and may include at least one of mutated target-specific nucleases that have been altered to eliminate endonuclease activity while retaining nickase activity, and mutated forms that have lost both endonuclease activity and nickase activity.

In the case of having nickase activity, either simultaneously with or sequentially, regardless of the order, with base conversion by the cytosine deaminase (e.g., cytosine to uridine conversion), a nick may be introduced to the strand where the base conversion occurs or the opposite strand (e.g., the strand opposite the strand where base conversion occurs) (e.g., a nick is introduced at a position between the third nucleotide and the fourth nucleotide in a direction of the 5' end of the PAM sequence on the strand opposite to the strand where PAM is located). Such mutations (e.g., amino acid substitutions, etc.) may occur in a catalytically active domain (e.g., a RuvC catalytic domain in Cas9). Streptococcus pyogenes-derived Cas9 may include mutations in which at least one selected from the group consisting of catalytic aspartate residue (aspartic acid at position 10 (D10), etc.), glutamic acid at position 762 (E762), histidine at position 840 (H840), asparagine at position 854 (N854), asparagine at position 863 (N863), aspartic acid at position 986 (D986), and the like is substituted with any different amino acid. At this point, the substituted different amino acid may be alanine, but is not limited to alanine.

In some cases, the Streptococcus pyogenes-derived Cas9 protein may be mutated to recognize NGA (where N is any base selected from among A, T, G, and C), which is different from the PAM sequence (NGG) of wild-type Cas9, by substituting one or more of aspartic acid at position 1135 (D1135), arginine at position 1335 (R1335), and threonine at position 1337 (T1337), for example, all three, with different amino acids.

For example, in the amino acid sequences of the Streptococcus pyogenes-derived Cas9 protein, amino acid substitution may occur at:
(1) D10, H840, or D10 + H840;
(2) D1135, R1335, T1337, or D1135 + R1335 + T1337; or
(3) both residues (1) and (2).

The "different amino acid" refers to an amino acid selected from alanine, isoleucine, leucine, methionine, phenylalanine, proline, tryptophan, valine, asparagine, cysteine, glutamine, glycine, serine, threonine, tyrosine, aspartic acid, glutamic acid, arginine, histidine, lysine, and all known variants of these amino acids, excluding the amino acid originally found at the mutation site in the wild-type protein. For example, the "different amino acid" may be alanine, valine, glutamine, or arginine.

In some cases, guide RNA may be further included. The guide RNA may be at least one selected from the group consisting of, for example, CRISPR RNA (crRNA), trans-activating crRNA (tracrRNA), and single guide RNA (sgRNA), and specifically, the guide RNA may be a double-stranded crRNA:tracrRNA complex in which crRNA and tracrRNA are linked to each other, or a single-stranded guide RNA (sgRNA) in which crRNA or a portion thereof and tracrRNA or a portion thereof are linked via an oligonucleotide linker.

The present invention includes an adenine deaminase. The adenine deaminase may be selected from the group consisting of, for example, APOBEC1 (apolipoprotein B editing complex 1), AID (activation-induced deaminase), and tadA (tRNA-specific adenosine deaminase), and specifically, the adenine deaminase may be tadA (tRNA-specific adenosine deaminase). The adenine deaminase may be, for example, a deoxy-adenine deaminase as a variant of E. coli TadA.

In a specific embodiment according to the present invention, the deoxy-adenine deaminase was used as a variant of E. coli TadA, and specifically, the deoxy-adenine deaminase may be TadA8e or ABE 8.0.

In a structure in which the cytosine deaminase is included in split form and the DNA binding protein is a zinc finger protein with the N-terminus of the zinc finger protein (ZF-Left) bound to the C-terminus of the first split of the cytosine deaminase and the C-terminus of the zinc finger protein (ZF-Right) bound to the N-terminus of the second split of the cytosine deaminase (NC configuration), the adenine deaminase may bind to the C-terminus of the zinc finger protein (ZF-Left), the N-terminus or C-terminus of the first split of the cytosine deaminase, the N-terminus of the zinc finger protein (ZF-Right), or the N-terminus or C-terminus of the second split of the cytosine deaminase.

The adenine deaminase may bind to the C-terminus of the zinc finger protein (ZF-Left), the N-terminus or C-terminus of the first split of the cytosine deaminase, the N-terminus of the zinc finger protein (ZF-Right), or the N-terminus or C-terminus of the second split of the cytosine deaminase even in structures in which the C-terminus of the zinc finger protein (ZF-Left) is bound to the N-terminus of the first split of the cytosine deaminase and the C-terminus of the zinc finger protein (ZF-Right) is bound to the N-terminus of the second split of the cytosine deaminase (CC configuration); the C-terminus of the zinc finger protein (ZF-Left) is bound to the N-terminus of the first split of the cytosine deaminase and the N-terminus of the zinc finger protein (ZF-Right) is bound to the C-terminus of the second split of the cytosine deaminase (CN configuration); or the N-terminus of the zinc finger protein (ZF-Left) is bound to the C-terminus of the first split of the cytosine deaminase and the N-terminus of the zinc finger protein (ZF-Right) is bound to the C-terminus of the second split of the cytosine deaminase (NN configuration).

When the cytosine deaminase is included in split form and the DNA binding protein is TALE, a first TALE may bind to the first split of the cytosine deaminase and a second TALE may bind to the second split of the cytosine deaminase, forming respective structures of N'-TALE-first split DDDA-C' and N'-TALE-second split DDDA-C'. The adenine deaminase may bind to the N-terminus or C-terminus of the first split of the cytosine deaminase or to the N-terminus or C-terminus of the second split of the cytosine deaminase.

The present invention relates to a composition (uracil DNA-glycosylase inhibitor(UGI)-free) for A-to-G base editing in plant cell organelle DNA, including 1) a DNA binding protein, 2) a split double-stranded DNA-specific bacterial cytosine deaminase, and 3) a deoxyadenine deaminase derived from E. coli TadA, in which the DNA binding protein is a zinc finger protein (ZFP) or a transcription activator-like effector (TALE) array, and the split double-stranded DNA-specific bacterial cytosine deaminase is DddAtox derived from Burkholderia cenocepacia.

In a structure in which the cytosine deaminase is included in split form and the DNA binding protein is a zinc finger protein with the N-terminus of the zinc finger protein (ZF-Left) bound to the C-terminus of the first split of the cytosine deaminase and the C-terminus of the zinc finger protein (ZF-Right) bound to the N-terminus of the second split of the cytosine deaminase (NC configuration), the adenine deaminase may bind to the C-terminus of the zinc finger protein (ZF-Left), the N-terminus or C-terminus of the first split of the cytosine deaminase, the N-terminus of the zinc finger protein (ZF-Right), or the N-terminus or C-terminus of the second split of the cytosine deaminase.

The adenine deaminase may bind to the C-terminus of the zinc finger protein (ZF-Left), the N-terminus or C-terminus of the first split of the cytosine deaminase, the N-terminus of the zinc finger protein (ZF-Right), or the N-terminus or C-terminus of the second split of the cytosine deaminase even in structures in which the C-terminus of the zinc finger protein (ZF-Left) is bound to the N-terminus of the first split of the cytosine deaminase and the C-terminus of the zinc finger protein (ZF-Right) is bound to the N-terminus of the second split of the cytosine deaminase (CC configuration); the C-terminus of the zinc finger protein (ZF-Left) is bound to the N-terminus of the first split of the cytosine deaminase and the N-terminus of the zinc finger protein (ZF-Right) is bound to the C-terminus of the second split of the cytosine deaminase (CN configuration); or the N-terminus of the zinc finger protein (ZF-Left) is bound to the C-terminus of the first split of the cytosine deaminase and the N-terminus of the zinc finger protein (ZF-Right) is bound to the C-terminus of the second split of the cytosine deaminase (NN configuration).

When the cytosine deaminase is included in split form and the DNA binding protein is TALE, a first TALE may bind to the first split of the cytosine deaminase and a second TALE may bind to the second split of the cytosine deaminase, forming respective structures of N'-TALE-first split DDDA-C' and N'-TALE-second split DDDA-C'. The adenine deaminase may bind to the N-terminus or C-terminus of the first split of the cytosine deaminase or to the N-terminus or C-terminus of the second split of the cytosine deaminase.

The present invention relates to a composition for A-to-G base editing in a plant cell organelle, in which the DNA binding protein is a zinc finger protein or a TALE array, and the first and second splits derived from the cytosine deaminase are derived from bacteria and are specific to double-stranded DNA.

The present invention relates to a composition for A-to-G base editing in a plant cell organelle, in which the DNA binding protein is a zinc finger protein or a TALE array, the first and second splits derived from the cytosine deaminase are derived from bacteria and are specific to double-stranded DNA, the DNA binding protein is attached to the N-terminus of the first split, and the DNA binding protein is attached to the C-terminus of the adenine deaminase.

The present invention relates to a composition (uracil DNA-glycosylase inhibitor(UGI)-free) for A-to-G base editing in plant cell organelle DNA, including 1) a DNA binding protein, 2) a split double-stranded DNA-specific bacterial cytosine deaminase, and 3) a deoxyadenine deaminase derived from E. coli TadA, in which the DNA binding protein is a zinc finger protein (ZFP) or a transcription activator-like effector (TALE) array, and the split double-stranded DNA-specific bacterial cytosine deaminase is DddAtox derived from Burkholderia cenocepacia.

The present invention relates to a method for A-to-G base editing in a plant cell organelle, including treating a plant cell with a DNA binding protein or a nucleic acid encoding the same, a first split and a second split derived from a cytosine deaminase or a nucleic acid encoding the same, and an adenine deaminase or a nucleic acid encoding the same, in which the DNA binding protein is a zinc finger protein or a TALE array, and the first split and the second split derived from the cytosine deaminase are derived from bacteria and are specific to double-stranded DNA.

The present invention relates to a method for A-to-G base editing in a plant cell organelle, including treating a plant cell with a DNA binding protein or a nucleic acid encoding the same, a first split and a second split derived from a cytosine deaminase or a nucleic acid encoding the same, and an adenine deaminase or a nucleic acid encoding the same, in which the DNA binding protein is a zinc finger protein or a TALE array, the first split and the second split derived from the cytosine deaminase are of bacterial origin and are specific to double-stranded DNA, the DNA binding protein is attached to the N-terminus of the first split, and the DNA binding protein is attached to the C-terminus of the adenine deaminase.

The present invention relates to a method for A-to-G base editing in a plant cell organelle, including treating a plant cell with a DNA binding protein or a nucleic acid encoding the same, a first split and a second split derived from a cytosine deaminase or a nucleic acid encoding the same, and an adenine deaminase or a nucleic acid encoding the same, particularly with a composition for A-to-G base editing in a plant cell organelle including 1) a DNA binding protein, 2) a split double-stranded DNA-specific bacterial cytosine deaminase, and 3) a deoxyadenine deaminase derived from E. coli TadA, wherein the composition does not include UGI, the DNA binding protein is a zinc finger protein (ZFP) or a transcription activator-like effector (TALE) array, and the split double-stranded DNA-specific bacterial cytosine deaminase is DddAtox derived from Burkholderia cenocepacia.

Previously, TALED without UGI caused only A-to-G editing, not C-to-T editing, in animal cell mitochondria.

The present invention may further include a chloroplast transit peptide or a mitochondrial targeting signal (MTS).

For example, the chloroplast transit peptide (CTP) or mitochondrial targeting signal (MTS) binds and is delivered to chloroplasts and mitochondria in plant cells. When delivered to the chloroplasts and mitochondria, the remainder except for the N-terminal CTP or MTS protein is delivered as a preprotein into the chloroplasts and mitochondria. During the process of entering the chloroplasts and mitochondria, the transit protein moiety is detached, and a specific portion may be base-edited by targeting the chloroplasts and mitochondria.

Regarding the nucleic acid, "polynucleotide", "nucleotide", "nucleotide sequence", and "oligonucleotide" are used interchangeably. Polymeric forms of nucleotides of any length, deoxyribonucleotides or ribonucleotides, or analogues thereof may be included. Polynucleotides may have any three-dimensional structure and may perform any known or unknown function. A polynucleotide may include one or more modified nucleotides, such as methylated nucleotides and nucleotide analogues. Modification to the nucleotide structure is possible before or after assembly of the polymer.

The polynucleotide may be an RNA sequence, a DNA sequence, or a combination thereof (RNA-DNA hybrid sequence).

As a means for expressing the fusion protein, known expression vectors such as plasmid vectors, cosmid vectors, bacteriophage vectors, etc. may be used, and vectors may be easily prepared by those skilled in the art according to any known method using recombinant DNA technology.

The vector may be a plasmid vector or a viral vector, and specific examples of the viral vector may include, but are not limited to, adenovirus, adeno-associated virus, lentivirus, and retrovirus vectors.

A recombinant expression vector may contain a nucleic acid in a form suitable for expression of the nucleic acid in a host cell, which means that it contains at least one regulatory element that may be selected on a host cell basis so that the recombinant expression vector is used for expression, namely is operably linked to the nucleic acid sequence to be expressed.

Within the recombinant expression vector, "operably linked" means that the nucleotide sequence of interest is connected to the regulatory element in a manner that allows expression of the nucleotide sequence (e.g., in an in vitro transcription/translation system or in a host cell when the vector is introduced into the host cell).

The recombinant expression vector may include forms suitable for messenger RNA synthesis, including a T7 promoter, which means that it includes at least one regulatory element to enable in situ mRNA synthesis, namely messenger RNA synthesis by T7 polymerase.

The "regulatory element" may include a promoter, an enhancer, an internal ribosome entry site (IRES), and other expression control elements (e.g., transcription termination signals such as polyadenylation signals and poly-U sequences). The regulatory element includes elements that direct the induction or constitutive expression of a nucleotide sequence in many types of host cells, and elements that direct expression of a nucleotide sequence only in certain host cells (e.g., tissue-specific regulatory sequences). A tissue-specific promoter may direct expression primarily in a desired tissue of interest such as muscle, neuron, bone, skin, blood, a specific organ (e.g., liver, pancreas), or a specific cell type (e.g., lymphocyte). The regulatory element may also direct expression in a temporally-dependent manner, such as in a cell cycle-dependent or developmental stage-dependent manner, which may or may not be tissue- or cell-type specific.

In some cases, the vector includes at least one pol III promoter, at least one pol II promoter, at least one pol I promoter, or combinations thereof. Examples of the pol III promoter include, but are not limited to, U6 and H1 promoters. Examples of the pol II promoter include, but are not limited to, retroviral Rous sarcoma virus (RSV) LTR promoter (optionally with RSV enhancer), cytomegalovirus (CMV) promoter (optionally with CMV enhancer) (e.g., Boshart et al. (1985) Cell 41:521-530), SV40 promoter, dihydrofolate reductase promoter, β-actin promoter, phosphoglycerol kinase (PGK) promoter, and EF1α promoter.

The "regulatory element" includes enhancers such as WPRE; CMV enhancer; R-U5' segment in LTR of HTLV-I; SV40 enhancer; and an intronic sequence between exons 2 and 3 of rabbit β-globin. It will be appreciated by those skilled in the art that the design of the expression vector may depend on factors such as the choice of host cell to be transformed, the level of expression desired, and the like. The vector may be introduced into a host cell to produce a transcript, protein, or peptide including a fusion protein or peptide encoded by a nucleic acid as described herein (e.g., a clustered regularly interspaced short palindromic repeat (CRISPR) transcript, protein, enzyme, mutants thereof, fusion proteins thereof, etc.). Advantageous vectors include lentiviruses and adeno-associated viruses, and these types of vectors may also be selected to target a certain type of cell.

The vector may be delivered in vivo or into cells through local injection (e.g., direct injection into a lesion or target site), electroporation, lipofection, viral vector, nanoparticles, PTD (protein translocation domain) fusion protein method, etc.

The nucleic acid may be injected in the form of ribonucleic acid such as messenger ribonucleic acid mRNA, enabling gene base editing in cells, for example, in animal cells or plant cells, without limitation.

The nucleic acid according to the present invention may be in the form of mRNA, and when delivered in the form of mRNA, compared to delivery in the form of a vector using DNA, a transcription process into mRNA is unnecessary, so that gene editing may be initiated quickly. There is a high possibility of transient protein expression.

The inventors of the present application have ascertained that, when a cytosine base editor is introduced into a plant cell in the form of ribonucleic acid such as messenger ribonucleic acid, for the purpose of editing plant cell organelle genes, the off-target effect is reduced compared to when it is delivered by a plasmid. It was demonstrated for the first time that, when a cytosine base editor is transformed into plant cells in the form of mRNA, it has an advantage in off-target effects compared to plasmids in the editing of plant cell organelle genes.

The mRNA may be delivered directly or through a carrier. In some cases, the mRNA of the nuclease and/or the cleavage factor may be chemically modified or directly delivered in the form of synthetic self-replicative RNA.

Methods of delivering mRNA molecules to cells in vitro or in vivo may be contemplated, including methods of delivering mRNA to cells or methods of delivering mRNA to cells of organisms such as humans or animals in vivo. For example, mRNA molecules may be delivered to cells, including lipids (e.g., liposomes, micelles, etc.), nanoparticles or nanotubes, cationic compounds (e.g., polyethyleneimine or PEI), or cationic compounds (e.g., polyethyleneimine or PEI). In some cases, biolistic methods which use techniques such as a gene gun or a biolistic particle delivery system, may be used for delivering mRNA into cells.

Examples of the carrier may include, but are not limited to, a cell penetrating peptide (CPP), nanoparticles, and a polymer.

The CPP is a short peptide that facilitates cellular uptake of various molecular cargoes (from nanoscale particles to small chemical molecules and large fragments of DNA).

Regarding the nanoparticles, the composition according to the present invention may be delivered via polymer nanoparticles, metal nanoparticles, metal/inorganic nanoparticles, or lipid nanoparticles. The polymer nanoparticles may be, for example, DNA nanoclews, yarn-like DNA nanoparticles that are synthesized by rolling circle amplification. DNA nanoclews, yarn-like DNA nanoparticles, may be loaded with mRNA and coated with PEI to improve endosomal escape capacity. These complexes bind to cell membranes, are internalized, and then translocate to the nucleus through endosome escape, where they may be delivered.

Regarding the metal nanoparticles, gold particles may be connected and complexed with a cationic endosomal disruptive polymer and thus delivered to cells. Examples of the cationic endosomal disruptive polymer may include polyethyleneimine, poly(arginine), poly(lysine), poly(histidine), poly-[2-{(2-aminoethyl)amino}-ethyl-aspartamide] (pAsp(DET)), block copolymer of poly(ethylene glycol) (PEG) and poly(arginine), block copolymer of PEG and poly(lysine), and block copolymer of PEG and poly{N-[N-(2-aminoethyl)-2-aminoethyl]aspartamide} (PEG-pAsp(DET)).

Regarding the metal/inorganic nanoparticles, mRNA may be encapsulated through, for example, ZIF-8 (zeolitic imidazolate framework-8).

In some cases, the mRNA may be negatively charged and coupled with cationic materials to form nanoparticles, which may penetrate cells through receptor-mediated endocytosis or phagocytosis.

Examples of the cationic polymer may include polyallylamine (PAH); polyethyleneimine (PEI); poly(L-lysine) (PLL); poly(L-arginine) (PLA); polyvinylamine homopolymers or copolymers; poly(vinylbenzyl-tri-C1-C4-alkylammonium salts); polymers of aliphatic or alicyclic dihalides and aliphatic N,N,N',N'-tetra-C1-C4-alkyl-alkylenediamines; poly(vinylpyridine) or poly(vinylpyridinium salt); poly(N,N-diallyl-N,N-di-C1-C4-alkylammonium halide); homopolymers or copolymers of quaternized di-C1-C4-alkyl-aminoethyl acrylates or methacrylates; POLYQUAD^{™}; polyaminoamide, and the like.

The cationic lipids may include cationic liposomal formulations. The lipid bilayer of liposomes may protect encapsulated nucleic acids from degradation and may prevent specific neutralization by antibodies capable of binding to nucleic acids. During endosomal maturation, endosome membranes and liposomes are fused, enabling efficient endosome escape of cationic lipid-nucleases. Examples of the cationic lipids may include polyethyleneimine, starburst polyamidoamine (PAMAM) dendrimers, Lipofectin (combination of DOTMA and DOPE), lipofectase, LIPOFECTAMINE^{®} (e.g., Lipofectamine^{®} 2000, Lipofectamine^{®} 3000, Lipofectamine^{®} RNAiMAX, Lipofectamine^{®} LTX), SAINT-RED (Synvolux Therapeutics, Groningen, Netherlands), DOPE, Cytopectin (Gilead Sciences, Foster City, California), and Eupectin (JBL, San Luis Obispo, California). Representative cationic liposomes may be prepared from N-[1-(2,3-dioleyloxy)propyl]-N,N,N-trimethylammonium chloride (DOTMA), N-[1-(2,3-dioleyloxy)propyl]-N,N,N-trimethylammonium methylsulfate (DOTAP), 3β-[N-(N',N'-dimethylaminoethane)carbamoyl]cholesterol (DC-Chol), 2,3-dioleyloxy-N-[2-(sperminecarboxamido)ethyl]-N,N-dimethyl-1-propanaminium trifluoroacetate (DOSPA), 1,2-dimyristyloxypropyl-3-dimethyl-hydroxyethyl ammonium bromide, or dimethyldioctadecylammonium bromide (DDAB).

Regarding the lipid nanoparticles, delivery is possible using liposomes as carriers. Liposomes are spherical vesicular structures composed of a unilamellar or multilamellar lipid bilayer surrounding an inner aqueous compartment and a relatively impermeable outer lipophilic phospholipid bilayer. Liposomal formulations may primarily contain natural phospholipids and lipids such as 1,2-distearoyl-sn-glycero-3-phosphatidylcholine (DSPC), sphingomyelin, phosphatidylcholine, or monosialoganglioside. In some cases, cholesterol or 1,2-dioleoyl-sn-glycero-3-phosphoethanolamine (DOPE) may be added to the lipid membrane in order to resolve instability in plasma. The addition of cholesterol reduces the rapid release of encapsulated bioactive compounds into plasma or 1,2-dioleoyl-sn-glycero-3-phosphoethanolamine (DOPE) serves to increase stability.

The composition may be delivered to plant cells through:
bombardment using a gene gun;
protoplast transfection mediated by polyethylene glycol (PEG);
protoplast transfection via electroporation; or
protoplast injection via microinjection.

The polynucleotide sequence encoding the fusion protein according to the present invention may be an RNA sequence, a DNA sequence, or a combination thereof (RNA-DNA hybrid sequence).

The nucleic acid may be delivered to plant cells through:
transformation using Agrobacterium, such as Agrobacterium tumefaciens, Agrobacterium rhizogene, etc.
   - binary vector,
   - Viral vector: Geminivirus, tobacco rattle virus (TRV), tomato mosaic virus (ToMV), foxtail mosaic virus (FoMV), barley yellow striate mosaic virus (BYSMV), Sonchus yellow net rhabdovirus (SYNV), etc.;
viral transfection;
bombardment using a gene gun;
protoplast transfection mediated by polyethylene glycol (PEG);
protoplast transfection via electroporation; or
protoplast injection via microinjection.

Examples of the virus may include a viral vector: geminivirus, tobacco rattle virus (TRV), tomato mosaic virus (ToMV), foxtail mosaic virus (FoMV), barley yellow striate mosaic virus (BYSMV), Sonchus yellow net rhabdovirus (SYNV), etc.

The vector may be delivered into cells through local injection (e.g., direct injection into a lesion or target site), electroporation, lipofection, viral vector, nanoparticles, PTD (protein translocation domain) fusion protein method, etc.

The present invention relates to a method for editing a base of plant cell organelle DNA, including treating a plant cell with the composition.

The present invention relates to an atrazine herbicide-resistant plant in which the base of the chloroplast psbA gene including the GTTGAAAGC sequence is edited by the method described above.

The present invention relates to a spectinomycin-resistant plant in which the base of 16s rRNA including the CGTCATCCTCA sequence is edited by the method described above.

The present invention relates to a plant in which the base of the atp6 gene is edited by the method described above.

The present invention relates to a plant having an albino phenotype in which the base of the psaA gene including ATG is edited by the method described above.

By base-editing phenylalanine, which is the 295th amino acid of psbA in chloroplast DNA, to serine, plants resistant to atrazine which is one of the herbicides may be developed. Spectinomycin-resistant plants and albino-type plants may be developed through base editing of 16s rRNA and psaA, as well as psbA in chloroplasts.

Since base editing of the RuBisCO large unit gene involved in photosynthesis is possible, it is expected that plant production will increase by controlling photosynthetic efficiency or that plants with enhanced carbon dioxide absorption efficiency can be developed.

The present invention relates to a plant in which the base of a RuBisCO (ribulose bisphosphate carboxylase) coding gene is edited to by the method described above. Since base editing of the RuBisCO large unit involved in photosynthesis is possible, it is expected that plant production will increase by controlling photosynthetic efficiency.

The base sequences of chloroplasts have high homology among plants, so not only the lettuce (Lactuca sativa cv. Cheongchima) used in the present invention, but also plants such as rice, wheat, potatoes, tomatoes and the like may be developed to be resistant to atrazine.

Plasmids were delivered into lettuce protoplasts, and adenine base editing was confirmed at the target site within the atp6 gene in mitochondrial DNA after seven days.

### Examples

Hereinafter, the present invention will be described in more detail through the following examples. These examples are merely set forth to illustrate the present invention and are not to be construed as limiting the scope of the present invention, as will be apparent to those skilled in the art.

### Example 1.

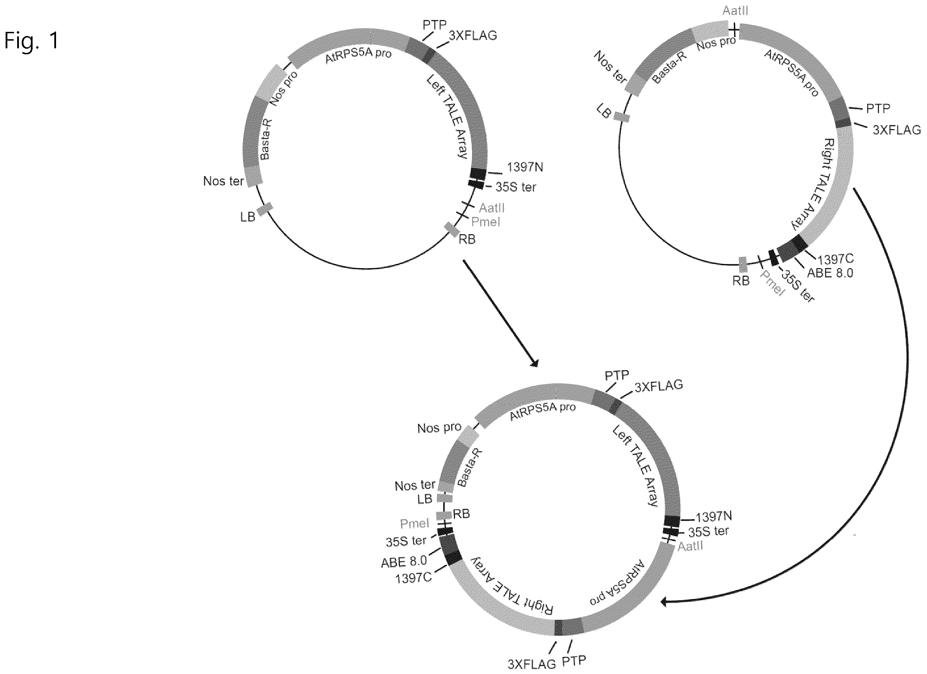

A schematic diagram of DNA cloning for producing transgenic plants using Agrobacterium is shown in FIG. 1. To clone a TALE pair with repetitive sequences into a single vector, RPS5A promoter-PTP-3xFlag-Left TALE-1397N and RPS5A promoter-PTP-3xFlag-Right TALE-1397C-ABE8.0 were cloned into separate vectors with different positions for Aat II and Pme I restriction sites. After digesting the two plasmids with Aat II and Pme I, respectively, RPS5A promoter-PTP-3xFlag-Right TALE-1397C-ABE8.0-35S terminator was ligated into the plasmid containing Left TALE, thereby cloning all components into a single plasmid. FIG. 1 is merely a schematic diagram, and it is possible to clone 1397C-ABE8.0 into Left TALE, while cloning 1397N into Right TALE.

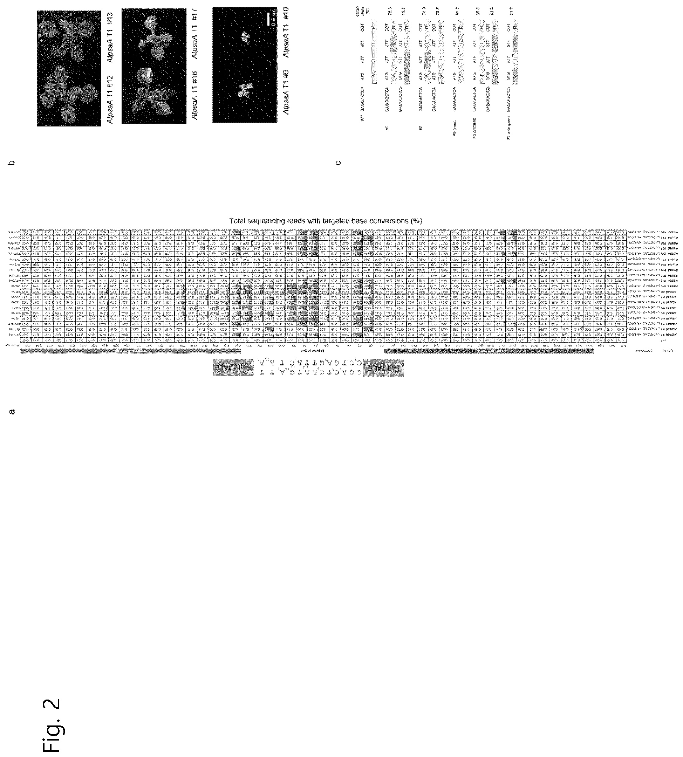

Through Agrobacterium-mediated transformation, 20, 6, and 37 first-generation transgenic plants targeting psaA, rbcL, and rrn16S genes in the chloroplasts of Arabidopsis thaliana were obtained, respectively. FIG. 2 illustrates transgenic plants targeting the psaA gene, showing (a) base editing efficiency and positions in each plant; (b) the plant phenotype; and (c) changes in amino acid sequences caused by DNA base editing. According to a in FIG. 2, it may be confirmed that C-12, C-11, and C2 (denoted as G2) among the base sequences are edited.

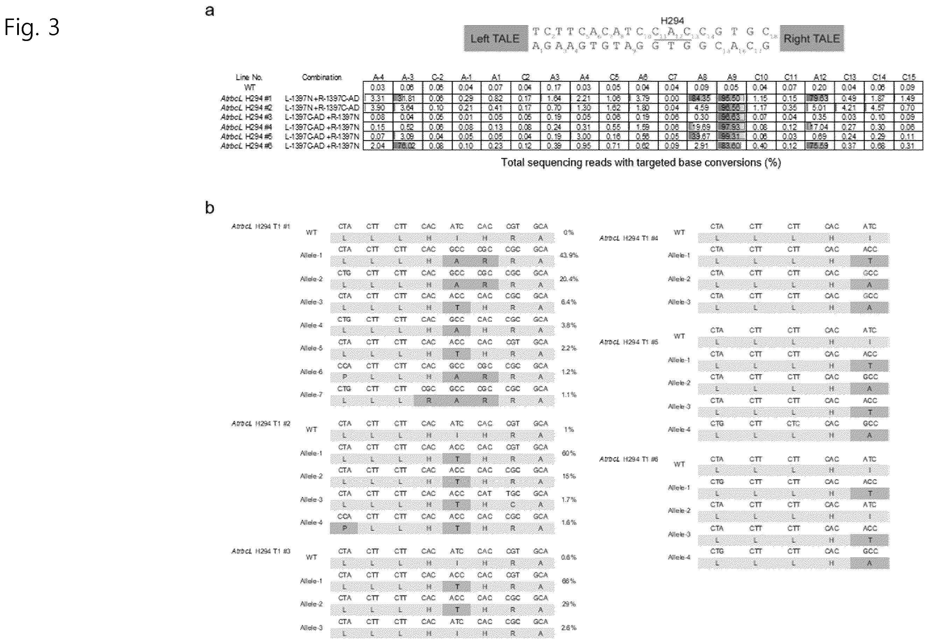

FIG. 3 illustrates transgenic plants targeting the rbcL gene, showing (a) base editing efficiency and positions in each plant; and (b) changes in amino acid sequences caused by DNA base editing.

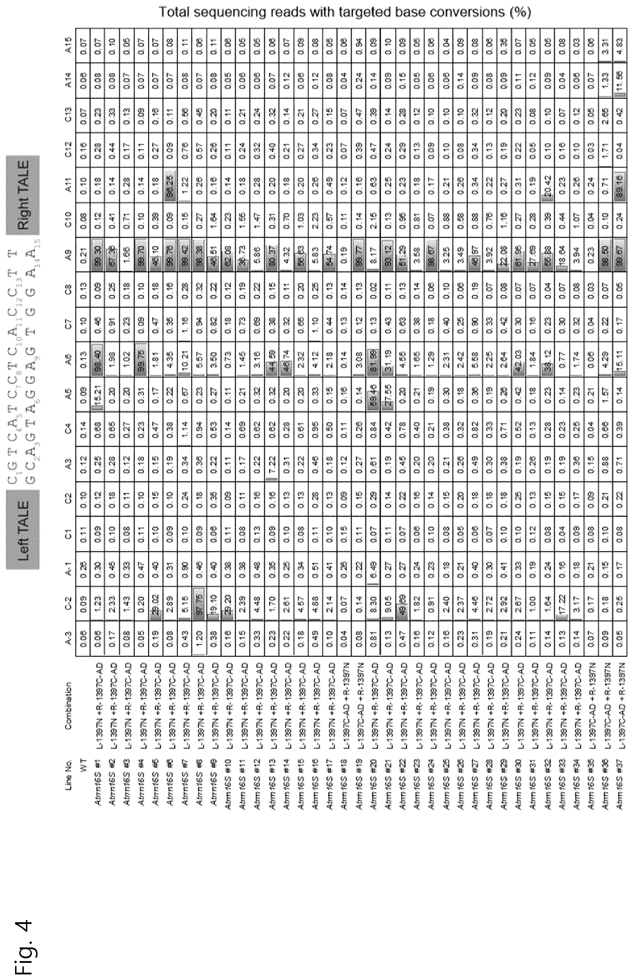

The base editing efficiency and positions in transgenic plants targeting the rrn16S gene are shown in FIG. 4.

FIG. 5 illustrates the base editing efficiency and phenotype of first-generation transgenic plants targeting the psaA, rbcL, and rrn16S genes in Arabidopsis chloroplasts, showing: (a) base editing efficiency and positions of psaA #1, #2, and #3, where not only base editing of adenine but also base editing of cytosine occur simultaneously; (b) phenotype of psaA #3, exhibiting green, chimeric, and pale green traits; (c) the base editing efficiency, positions, and phenotype of rbcL #1 and #2; and (d) base editing efficiency of first-generation transgenic plants targeting the rrn16S gene.

It may be confirmed that C-2 in FIG. 4 and C2 and C-2 in a and d of FIG. 5 were successfully edited.

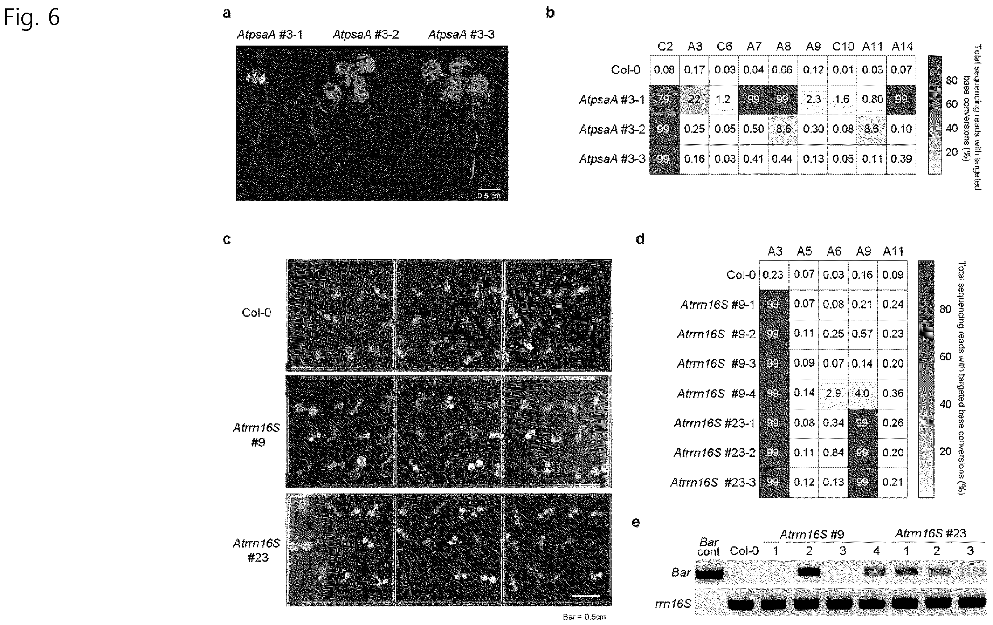

FIG. 6 illustrates the base editing efficiency and phenotype of second-generation transgenic plants, showing: (a) the phenotype of second-generation psaA #3 transgenic plants; (b) base editing efficiency and positions in psaA #3; (c) the phenotype of second-generation rrn16S transgenic plants exhibiting spectinomycin resistance; (d) base editing efficiency and positions in rrn16S transgenic plants; and (e) PCR analysis confirming the presence or absence of foreign genes in transgenic plants, which confirmed that rrn16S #9-1 and #9-3 lack foreign genes.

FIG. 6 confirms that chloroplast base editing is inherited to the next generation, and in (b), it was confirmed that both A-to-G and C-to-T base editing are inherited together.

Based on this, it was confirmed that, unlike in animals, A-to-G and C-to-T base editing occur together in plants.

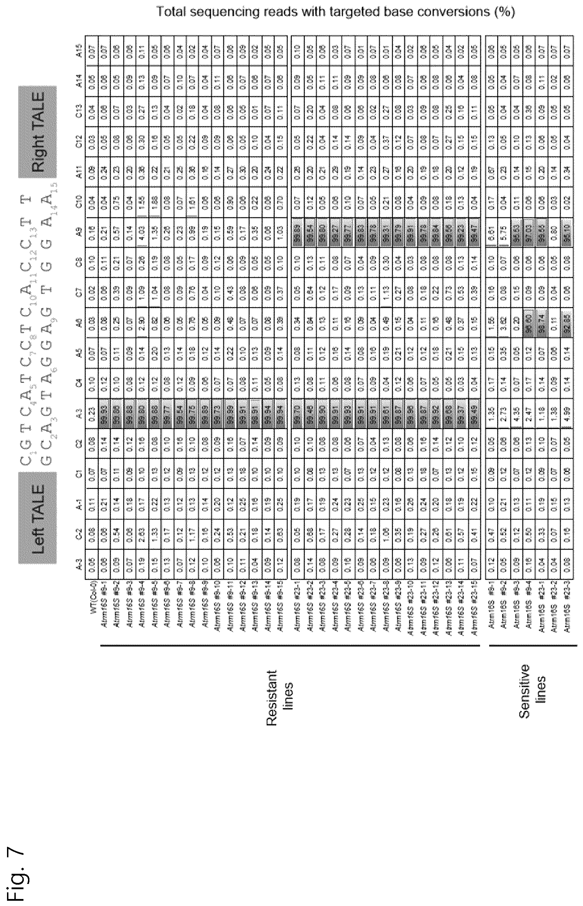

The base editing positions and efficiency in plants resistant and sensitive to spectinomycin among the second-generation rrn16S transgenic plants are shown in FIG. 7.

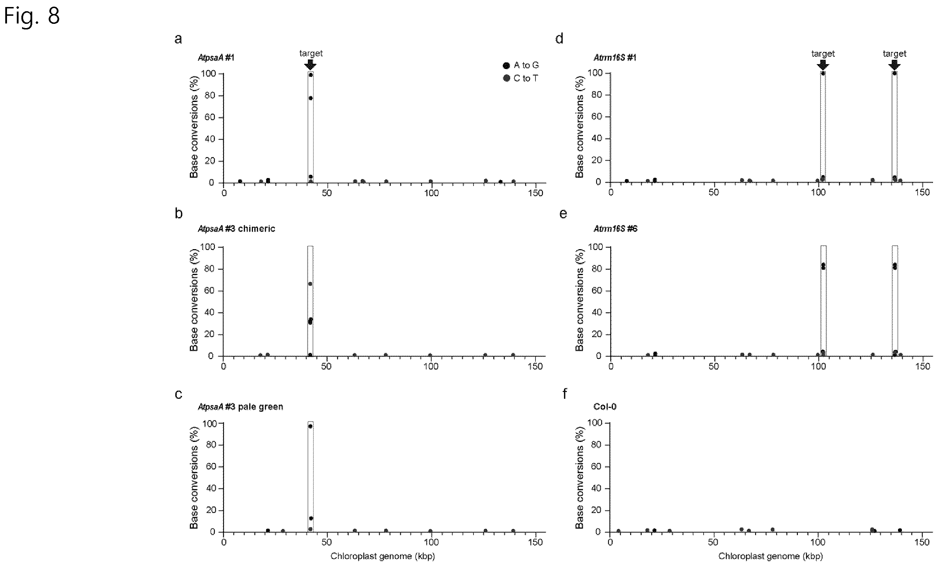

According to FIG. 8, whole chloroplast genome analysis was conducted to examine off-target base mutations in (a) psaA #1, (b) psaA #3 chimeric, (c) psaA #3 pale green, (d) rrn16S #1, (e) rrn16S #6, and (f) wild-type Col-0. No distinct off-target base mutations were observed in transgenic plants compared to the wild type.

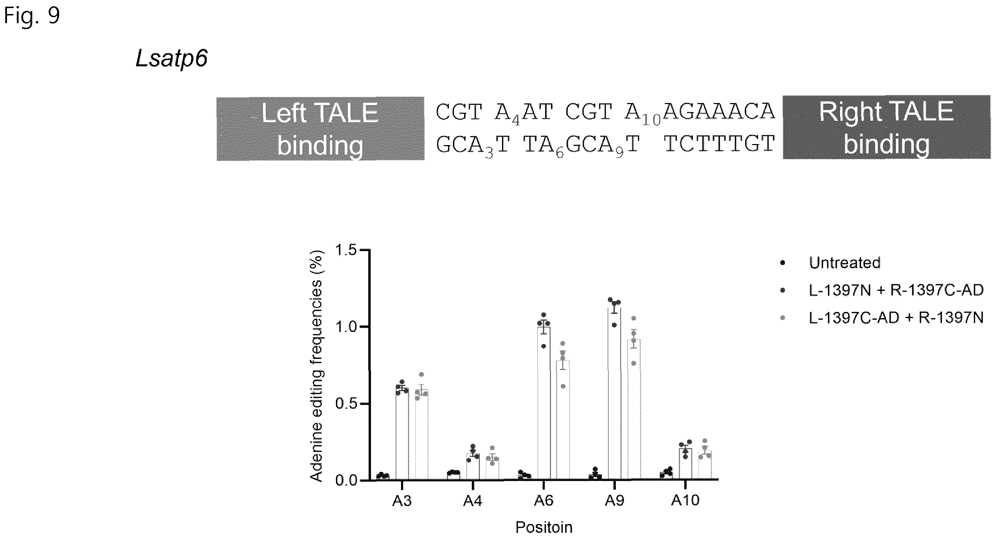

According to FIG. 9, the base editing of the lettuce mitochondrial gene atp6 was shown to be efficient. A total of 30 µg was delivered to lettuce protoplasts, with 15 µg of PcUBi promoter-MTS-3xFlag-Left TALE-1397N-Pea3A terminator and PcUBi promoter-MTS-3xFlag-Right TALE-1397C-ABE8.0-Pea3A terminator, along with 15 µg of PcUBi promoter-MTS-3xFlag-Left TALE-1397C-ABE8.0-Pea3A terminator and PcUBi promoter-MTS-3xFlag-Right TALE-1397N-Pea3A terminator.

The sequences of each component used in the examples are as follows.
CTS (or PTP: SEQ ID NO: 16)
   MDSQLVLSLKLNPSFTPLSPLFPFTPCSSFSPSLRFSSCYSRRLYSPVTVYAAK
MTS (SEQ ID NO: 17)
   MFKQASRLSRSVAAAASSKSVTTRAFSTELPSTLDS
NTD (TALE N-term SEQ ID NO: 18) 
DLRTLGYSQQQQEKIKPKVRSTVAQHHEALVGHGFTHAHIVALSQHPAALGTVAVKYQDMIAA
LPEATHEAIVGVGKQWSGARALEALLTVAGELRGPPLQLDTGQLLKIAKRGGVTAVEAVHAWRNALTGAP
LN
CTD (TALE C-term SEQ ID NO: 19)
   LTPEQVVAIASNGGGKQALESIVAQLSRPDPALAALTNDHLVALACLGGRPALDAVKKGLG
AD (ABE8.0 or TadA8e: (SEQ ID NO: 20) 
SEVEFSHEYWMRHALTLAKRARDEREVPVGAVLVLNNRVIGEGWNRAIGLHDPTAHAEIMAL
RQGGLVMQNYRLIDATLYVTFEPCVMCAGAMIHSRIGRVVFGVRNSKRGAAGSLMNVLNYPGMNHRV
EITEGILADECAALLCDFYRMPRQVFNAQKKAQSSIN
Linker 1 (TALE array - Linker 1-DddAtox)
   GS
Linker 2 (DddAtox-Linker 2-AD)
   SGSETPGTSESATPES
DddAtox 1397N (SEQ ID NO: 21) 
GSGSYALGPYQISAPQLPAYNGQTVGTFYYVNDAGGLESKVFSSGGTPPYPNYANAGHVEGQS
ALFMRDNGISEGLVFHNNPEGTCGFCVNMTETLLPENAKMTVVPPEG
DddAtox 1397C (SEQ ID NO: 22)
   GSAIPVKRGATGETKVFTGNNSNSPKSPTKGGC
psbA Left TALE repeat (SEQ ID NO: 23) 
LTPAQVVAIASNGGGKQALETVQRLLPVLCQAHGLTPAQVVAIASNNGGKQALETVQRLLPVLC
QDHGLTPAQVVAIASNIGGKQALETVQRLLPVLCQDHGLTPDQVVAIASNIGGKQALETVQRLLPVLCQA
HGLTPDQVVAIASNIGGKQALETVQRLLPVLCQDHGLTPDQVVAIASNGGGKQALETVQRLLPVLCQAHG
LTPAQVVAIASNGGGKQALETVQRLLPVLCQAHGLTPAQVVAIASNNGGKQALETVQRLLPVLCQDHGLT
PAQVVAIASNIGGKQALETVQRLLPVLCQAHGLTPDQVVAIASNIGGKQALETVQRLLPVLCQAHGLTPDQ
VVAIASNIGGKQALETVQRLLPVLCQAHGLTPAQVVAIASHDGGKQALETVQRLLPVLCQAHGLTPAQVVA
IASHDGGKQALETVQRLLPVLCQDHGLTPDQVVAIASNIGGKQALETVQRLLPVLCQDHGLTPAQVVAIAS
NGGGKQALETVQRLLPVLCQAHGLTPEQVVAIASNGGGKQALETVQRLLPVLCQAHGLTPDQVVAIASN
GGGKQALETVQRLLPVLCQAHG
psbA Right TALE repeat (SEQ ID NO: 24) 
LTPDQVVAIASNNGGKQALETVQRLLPVLCQAHGLTPAQVVAIASHDGGKQALETVQRLLPVLC
QDHGLTPDQVVAIASNGGGKQALETVQRLLPVLCQAHGLTPEQVVAIASNGGGKQALETVQRLLPVLCQ
AHGLTPDQVVAIASNGGGKQALETVQRLLPVLCQDHGLTPDQVVAIASNIGGKQALETVQRLLPVLCQDH
GLTPAQVVAIASNNGGKQALETVQRLLPVLCQDHGLTPAQVVAIASNNGGKQALETVQRLLPVLCQDHGL
TPDQVVAIASNGGGKQALETVQRLLPVLCQAHGLTPDQVVAIASNIGGKQALETVQRLLPVLCQDHGLTP
AQVVAIASNGGGKQALETVQRLLPVLCQAHGLTPAQVVAIASHDGGKQALETVQRLLPVLCQDHGLTPAQ
VVAIASNIGGKQALETVQRLLPVLCQDHGLTPDQVVAIASNNGGKQALETVQRLLPVLCQDHGLTPEQVV
AIASHDGGKQALETVQRLLPVLCQAHGLTPEQVVAIASNIGGKQALETVQRLLPVLCQAHGLTPAQVVAIA
SHDGGKQALETVQRLLPVLCQDHG
rrn16S Left TALE repeat (SEQ ID NO: 25) 
LTPAQVVAIASNIGGKQALETVQRLLPVLCQDHGLTPDQVVAIASNIGGKQALETVQRLLPVLCQ
DHGLTPDQVVAIASNNGGKQALETVQRLLPVLCQAHGLTPAQVVAIASNNGGKQALETVQRLLPVLCQA
HGLTPDQVVAIASNNGGKQALETVQRLLPVLCQDHGLTPEQVVAIASNNGGKQALETVQRLLPVLCQAH
GLTPAQVVAIASHDGGKQALETVQRLLPVLCQDHGLTPDQVVAIASNIGGKQALETVQRLLPVLCQDHGLT
PAQVVAIASNGGGKQALETVQRLLPVLCQAHGLTPAQVVAIASNNGGKQALETVQRLLPVLCQDHGLTPA
QVVAIASNIGGKQALETVQRLLPVLCQAHGLTPEQVVAIASNGGGKQALETVQRLLPVLCQAHGLTPAQV
VAIASNNGGKQALETVQRLLPVLCQDHGLTPAQVVAIASNIGGKQALETVQRLLPVLCQAHGLTPDQVVAI
ASHDGGKQALETVQRLLPVLCQAHGLTPEQVVAIASNGGGKQALETVQRLLPVLCQAHGLTPAQVVAIAS
 NGGGKQALETVQRLLPVLCQAHGLTPDQVVAIASNNGGKQALETVQRLLPVLCQDHG
rrn16S Right TALE repeat (SEQ ID NO: 26) 
LTPDQVVAIASNNGGKQALETVQRLLPVLCQAHGLTPDQVVAIASHDGGKQALETVQRLLPVLC
QDHGLTPDQVVAIASHDGGKQALETVQRLLPVLCQAHGLTPAQVVAIASNNGGKQALETVQRLLPVLCQ
DHGLTPAQVVAIASNNGGKQALETVQRLLPVLCQDHGLTPDQVVAIASNGGGKQALETVQRLLPVLCQA
HGLTPAQVVAIASNNGGKQALETVQRLLPVLCQDHGLTPAQVVAIASNIGGKQALETVQRLLPVLCQAHG
LTPEQVVAIASNGGGKQALETVQRLLPVLCQAHGLTPAQVVAIASNIGGKQALETVQRLLPVLCQDHGLTP
DQVVAIASNIGGKQALETVQRLLPVLCQDHGLTPDQVVAIASNNGGKQALETVQRLLPVLCQAHGLTPAQ
VVAIASHDGGKQALETVQRLLPVLCQDHGLTPDQVVAIASHDGGKQALETVQRLLPVLCQAHGLTPEQVV
AIASNNGGKQALETVQRLLPVLCQAHGLTPDQVVAIASNNGGKQALETVQRLLPVLCQDHGLTPAQVVAI
ASNIGGKQALETVQRLLPVLCQAHGLTPEQVVAIASNNGGKQALETVQRLLPVLCQAHG
psaA Left TALE repeat (SEQ ID NO: 27) 
LTPAQVVAIASHDGGKQALETVQRLLPVLCQAHGLTPAQVVAIASNGGGKQALETVQRLLPVLC
QDHGLTPDQVVAIASNNGGKQALETVQRLLPVLCQDHGLTPAQVVAIASNNGGKQALETVQRLLPVLCQ
DHGLTPEQVVAIASNGGGKQALETVQRLLPVLCQAHGLTPAQVVAIASNGGGKQALETVQRLLPVLCQDH
GLTPAQVVAIASHDGGKQALETVQRLLPVLCQDHGLTPDQVVAIASHDGGKQALETVQRLLPVLCQAHGL
TPEQVVAIASNNGGKQALETVQRLLPVLCQAHGLTPAQVVAIASNNGGKQALETVQRLLPVLCQAHGLTP
DQVVAIASHDGGKQALETVQRLLPVLCQDHGLTPAQVVAIASNNGGKQALETVQRLLPVLCQAHGLTPD
QVVAIASNIGGKQALETVQRLLPVLCQAHGLTPDQVVAIASNIGGKQALETVQRLLPVLCQAHGLTPAQVV
AIASHDGGKQALETVQRLLPVLCQAHGLTPEQVVAIASNNGGKQALETVQRLLPVLCQAHGLTPDQVVAI
ASNIGGKQALETVQRLLPVLCQDHGLTPDQVVAIASNIGGKQALETVQRLLPVLCQAHG
psaA Right TALE repeat (SEQ ID NO: 28) 
LTPEQVVAIASNNGGKQALETVQRLLPVLCQAHGLTPDQVVAIASNGGGKQALETVQRLLPVLC
QDHGLTPAQVVAIASNNGGKQALETVQRLLPVLCQAHGLTPEQVVAIASNGGGKQALETVQRLLPVLCQA
HGLTPAQVVAIASNGGGKQALETVQRLLPVLCQAHGLTPDQVVAIASNNGGKQALETVQRLLPVLCQDH
GLTPAQVVAIASNGGGKQALETVQRLLPVLCQDHGLTPAQVVAIASHDGGKQALETVQRLLPVLCQAHGL
TPEQVVAIASHDGGKQALETVQRLLPVLCQAHGLTPAQVVAIASNNGGKQALETVQRLLPVLCQAHGLTP
DQVVAIASNNGGKQALETVQRLLPVLCQDHGLTPAQVVAIASNIGGKQALETVQRLLPVLCQAHGLTPAQ
VVAIASNIGGKQALETVQRLLPVLCQDHGLTPDQVVAIASNIGGKQALETVQRLLPVLCQDHGLTPDQVVA
IASNNGGKQALETVQRLLPVLCQAHGLTPAQVVAIASNIGGKQALETVQRLLPVLCQAHGLTPAQVVAIAS
NNGGKQALETVQRLLPVLCQDHGLTPAQVVAIASNNGGKQALETVQRLLPVLCQAHG
rbc L Left TALE repeat (SEQ ID NO: 29) 
LTPDQVVAIASNNGGKQALETVQRLLPVLCQAHGLTPDQVVAIASHDGGKQALETVQRLLPVLC
QDHGLTPDQVVAIASHDGGKQALETVQRLLPVLCQAHGLTPDQVVAIASNNGGKQALETVQRLLPVLCQ
DHGLTPAQVVAIASNIGGKQALETVQRLLPVLCQAHGLTPEQVVAIASNNGGKQALETVQRLLPVLCQAH
GLTPAQVVAIASNIGGKQALETVQRLLPVLCQDHGLTPAQVVAIASNGGGKQALETVQRLLPVLCQDHGLT
 
PDQVVAIASNIGGKQALETVQRLLPVLCQAHGLTPEQVVAIASNIGGKQALETVQRLLPVLCQAHGLTPAQ
VVAIASNGGGKQALETVQRLLPVLCQAHGLTPAQVVAIASNNGGKQALETVQRLLPVLCQDHGLTPDQV
VAIASNNGGKQALETVQRLLPVLCQAHGLTPDQVVAIASHDGGKQALETVQRLLPVLCQDHGLTPDQVV
AIASHDGGKQALETVQRLLPVLCQAHGLTPEQVVAIASNGGGKQALETVQRLLPVLCQAHGLTPDQVVAI
ASNIGGKQALETVQRLLPVLCQAHGLTPAQVVAIASHDGGKQALETVQRLLPVLCQDHG
rbc L Right TALE repeat (SEQ ID NO: 30) 
LTPDQVVAIASNIGGKQALETVQRLLPVLCQDHGLTPAQVVAIASNGGGKQALETVQRLLPVLC
QAHGLTPAQVVAIASHDGGKQALETVQRLLPVLCQDHGLTPDQVVAIASNIGGKQALETVQRLLPVLCQA
HGLTPDQVVAIASNIGGKQALETVQRLLPVLCQDHGLTPDQVVAIASNGGGKQALETVQRLLPVLCQAHG
LTPDQVVAIASNIGGKQALETVQRLLPVLCQAHGLTPDQVVAIASNIGGKQALETVQRLLPVLCQAHGLTP
AQVVAIASHDGGKQALETVQRLLPVLCQAHGLTPAQVVAIASNIGGKQALETVQRLLPVLCQDHGLTPDQ
VVAIASNNGGKQALETVQRLLPVLCQDHGLTPEQVVAIASHDGGKQALETVQRLLPVLCQAHGLTPEQVV
AIASNNGGKQALETVQRLLPVLCQAHGLTPDQVVAIASNGGGKQALETVQRLLPVLCQDHGLTPAQVVAI
ASNNGGKQALETVQRLLPVLCQAHGLTPAQVVAIASHDGGKQALETVQRLLPVLCQDHGLTPDQVVAIAS
NIGGKQALETVQRLLPVLCQDHGLTPAQVVAIASNGGGKQALETVQRLLPVLCQAHG
atp6 Left TALE repeat (SEQ ID NO: 31) 
LTPDQVVAIASNNGGKQALETVQRLLPVLCQAHGLTPAQVVAIASHDGGKQALETVQRLLPVLC
QDHGLTPDQVVAIASNGGGKQALETVQRLLPVLCQAHGLTPAQVVAIASHDGGKQALETVQRLLPVLCQ
DHGLTPEQVVAIASNIGGKQALETVQRLLPVLCQAHGLTPAQVVAIASNIGGKQALETVQRLLPVLCQDHG
LTPEQVVAIASNNGGKQALETVQRLLPVLCQAHGLTPDQVVAIASNGGGKQALETVQRLLPVLCQDHGLT
PAQVVAIASNNGGKQALETVQRLLPVLCQAHGLTPEQVVAIASNNGGKQALETVQRLLPVLCQAHGLTPA
QVVAIASNNGGKQALETVQRLLPVLCQDHGLTPDQVVAIASHDGGKQALETVQRLLPVLCQDHGLTPAQ
VVAIASNGGGKQALETVQRLLPVLCQAHGLTPDQVVAIASNNGGKQALETVQRLLPVLCQAHGLTPAQVV
AIASNGGGKQALETVQRLLPVLCQDHGLTPEQVVAIASNGGGKQALETVQRLLPVLCQAHGLTPAQVVAI
ASNNGGKQALETVQRLLPVLCQDHGLTPDQVVAIASNNGGKQALETVQRLLPVLCQDHG
atp6 Right TALE repeat (SEQ ID NO: 32) 
LTPDQVVAIASNIGGKQALETVQRLLPVLCQDHGLTPDQVVAIASHDGGKQALETVQRLLPVLC
QDHGLTPAQVVAIASNIGGKQALETVQRLLPVLCQAHGLTPAQVVAIASNNGGKQALETVQRLLPVLCQA
HGLTPDQVVAIASNNGGKQALETVQRLLPVLCQDHGLTPEQVVAIASNNGGKQALETVQRLLPVLCQAH
GLTPEQVVAIASNIGGKQALETVQRLLPVLCQAHGLTPAQVVAIASNGGGKQALETVQRLLPVLCQAHGLT
PAQVVAIASNNGGKQALETVQRLLPVLCQAHGLTPDQVVAIASNIGGKQALETVQRLLPVLCQAHGLTPA
QVVAIASNIGGKQALETVQRLLPVLCQDHGLTPAQVVAIASNIGGKQALETVQRLLPVLCQAHGLTPAQVV
AIASNGGGKQALETVQRLLPVLCQAHGLTPAQVVAIASNNGGKQALETVQRLLPVLCQDHGLTPAQVVAI
ASNIGGKQALETVQRLLPVLCQDHGLTPDQVVAIASNIGGKQALETVQRLLPVLCQAHGLTPAQVVAIASN
IGGKQALETVQRLLPVLCQDHGLTPAQVVAIASNIGGKQALETVQRLLPVLCQAHG
RPS5A promoter (SEQ ID NO: 41) 
CTCAACTTTTGATTCGCTATTTGCAGTGCACCTGTGGCGTTCATCACATCTTTTGTGACACTGT
TTGCACTGGTCATTGCTATTACAAAGGACCTTCCTGATGTTGAAGGAGATCGAAAGTAAGTAACTGCAC
GCATAACCATTTTCTTTCCGCTCTTTGGCTCAATCCATTTGACAGTCAAAGACAATGTTTAACCAGCTCC
GTTTGATATATTGTCTTTATGTGTTTGTTCAAGCATGTTTAGTTAATCATGCCTTTGATTGATCTTGAATAG
GTTCCAAATATCAACCCTGGCAACAAAACTTGGAGTGAGAAACATTGCATTCCTCGGTTCTGGACTTCT
GCTAGTAAATTATGTTTCAGCCATATCACTAGCTTTCTACATGCCTCAGGTGAATTCATCTATTTCCGTCTT
AACTATTTCGGTTAATTAAAGCACGAACACCATTACTGCATGTAGAAGCTTGATAAACTATCGCCACCAA
TTTATTTTTGTTGCGATATTGTTACTTTCCTCAGTATGCAGCTTTGAAAAGACCAACCCTCTTATCCTTTAA
CAATGAACAGGTTTTTAGAGGTAGCTTGATGATTCCTGCACATGTGATCTTGGCTTCAGGCTTAATTTTC
CAGGTAAAGCATTATGAGATACTCTTATATCTCTTACATACTTTTGAGATAATGCACAAGAACTTCATAAC
TATATGCTTTAGTTTCTGCATTTGACACTGCCAAATTCATTAATCTCTAATATCTTTGTTGTTGATCTTTGGT
AGACATGGGTACTAGAAAAAGCAAACTACACCAAGGTAAAATACTTTTGTACAAACATAAACTCGTTAT
CACGGAACATCAATGGAGTGTATATCTAACGGAGTGTAGAAACATTTGATTATTGCAGGAAGCTATCTCA
GGATATTATCGGTTTATATGGAATCTCTTCTACGCAGAGTATCTGTTATTCCCCTTCCTCTAGCTTTCAATT
TCATGGTGAGGATATGCAGTTTTCTTTGTATATCATTCTTCTTCTTCTTTGTAGCTTGGAGTCAAAATCGG
TTCCTTCATGTACATACATCAAGGATATGTCCTTCTGAATTTTTATATCTTGCAATAAAAATGCTTGTACCA
ATTGAAACACCAGCTTTTTGAGTTCTATGATCACTGACTTGGTTCTAACCAAAAAAAAAAAAATGTTTAA
TTTACATATCTAAAAGTAGGTTTAGGGAAACCTAAACAGTAAAATATTTGTATATTATTCGAATTTCACTC
ATCATAAAAACTTAAATTGCACCATAAAATTTTGTTTTACTATTAATGATGTAATTTGTGTAACTTAAGATA
AAAATAATATTCCGTAAGTTAACCGGCTAAAACCACGTATAAACCAGGGAACCTGTTAAACCGGTTCTTT
ACTGGATAAAGAAATGAAAGCCCATGTAGACAGCTCCATTAGAGCCCAAACCCTAAATTTCTCATCTATA
TAAAAGGAGTGACATTAGGGTTTTTGTTCGTCCTCTTAAAGCTTCTCGTTTTCTCTGCCGTCTCTCTCATT
CGCGCGACGCAAACGATCTTCAGGTGATCTTCTTTCTCCAAATCCTCTCTCATAACTCTGATTTCGTACTT
GTGTATTTGAGCTCACGCTCTGTTTCTCTCACCACAGCC
35S terminator (SEQ ID NO: 42) 
CGGCCATGCTAGAGTCCGCAAAAATCACCAGTCTCTCTACAAATCTATCTCTCTCTCTATTTT
TCTCCAGAATAATGTGTGAGTAGTTCCCAGATAAGGGAATTAGGGTTTCTTATAGGGTTTCGCTCATGTG
TTGAGCATATAAGAAACCCTTAGTATGTATTTGTATTTGTAAAATACTTCTATCAATAAAAATTTCTAATTCC
TAAAACCAAAATCCAGTGACCT
PcUbi promoter (SEQ ID NO: 43) 
CTAGCAACGATTGTACAATTGCTTCTTTAAAAAAGGAAGAAAGAAAGAAAAGAATCA
ACATCAGCGTTAACAAACGGCCCCGTTACGGCCCAAACGGTCATATAGAGTAACGGCGTTAAGCGTTG
AAAGACTCCTATCGAAATACGTAACCGCAAACGTGTCATAGTCAGATCCCCTCTTCCTTCACCGCCTCAA
ACACAAAAATAATCTTCTACAGCCTATATATACAACCCCCCCTTCTATCTCTCCTTTCTCACAATTCATCATC
TTTCTTTCTCTACCCCCAATTTTAAGAAATCCTCTCTTCTCCTCTTCATTTTCAAGGTAAATCTCTCTCTCTC
TCTCTCTCTCTGTTATTCCTTGTTTTAATTAGGTATGTATTATTGCTAGTTTGTTAATCTGCTTATCTTATGTA
TGCCTTATGTGAATATCTTTATCTTGTTCATCTCATCCGTTTAGAAGCTATAAATTTGTTGATTTGACTGTG
 
TATCTACACGTGGTTATGTTTATATCTAATCAGATATGAATTTCTTCATATTGTTGCGTTTGTGTGTACCAAT
CCGAAATCGTTGATTTTTTTCATTTAATCGTGTAGCTAATTGTACGTATACATATGGATCTACGTATCAATT
GTTCATCTGTTTGTGTTTGTATGTATACAGATCTGAAAACATCACTTCTCTCATCTGATTGTGTTGTTACAT
ACATAGATATAGATCTGTTATATCATTTTTTTTATTAATTGTGTATATATATATGTGCATAGATCTGGATTACA
TGATTGTGATTATTTACATGATTTTGTTATTTACGTATGTATATATGTAGATCTGGACTTTTTGGAGTTGTTG
ACTTGATTGTATTTGTGTGTGTATATGTGTGTTCTGATCTTGATATGTTATGTATGTGCAGC
Pea3A terminator (SEQ ID NO: 44)

CAGGCCTCCCAGCTTTCGTCCGTATCATCGGTTTCGACAACGTTCGTCAAGTTCAATGCATCAGTTT
CATTGCCCACACACCAGAATCCTACTAAGTTTGAGTATTATGGCATTGGAAAAGCTGTTTTCTTCTATCAT
TTGTTCTGCTTGTAATTTACTGTGTTCTTTCAGTTTTTGTTTTCGGACATCAAAATGCAAATGGATGGATA
AGAGTTAATAAATGATATGGTCCTTTTGTTCATTCTCAAATTATTATTATCTGTTGTTTTTACTTTAATGGGT
TGAATTTAAGTAAGAAAGGAACTAACAGTGTGATATTAAGGTGCAATGTTAGACATATAAAACAGTCTTT
CACCTCTCTTTGGTTATGTCTTGAATTGGTTTGTTTCTTCACTTATCTGTGTAATCAAGTTTACTATGAGTC
TATGATCAAGTAATTATGCAATCAAGTTAAGTACAGTATAGGCTT

### [Industrial Applicability]

The only base editing of plant cell organelle DNA only existed through deamination of cytosine to thymine using DddAtox. This invention expands the scope of DNA base editing in small organelles by base-editing adenine to guanine. A to G base editing and C to T base editing may occur simultaneously.

Having described specific parts of the present invention in detail above, it will be obvious to those skilled in the art that these specific descriptions are only preferred embodiments, and the scope of the present invention is not limited thereby. Accordingly, the substantial scope of the present invention will be defined by the appended claims and equivalents thereto.

### [Sequence List Free Text]

An electronic file is attached.

## Claims

1. A composition for editing organellar DNA in plant cells, comprising:
DNA binding protein or nucleic acid encoding the DNA binding protein;
cytosine deaminase, or first and second splits derived from cytosine deaminase, or nucleic acid encoding the same; and
adenine deaminase or nucleic acid encoding the adenine deaminase,
wherein each of the first and second splits independently binds to DNA binding protein, and
the composition simultaneously performs adenine (A)-to-guanine (G) editing and cytosine (C)-to-thymine (T) editing in plant organellar DNA.

2. The composition according to claim 1, wherein each of the first and second splits alone lacks cytosine deaminase activity.

3. The composition according to claim 1, wherein the cytosine deaminase is derived from a double-stranded DNA deaminase (DddA) or an orthologue thereof.

4. The composition according to claim 1, wherein the first split comprises the sequence from the N-terminus to at least one residue selected from the group consisting of G33, G44, A54, N68, G82, N98, and G108 of SEQ ID NO:1.

5. The composition according to claim 1, wherein the second split comprises the sequence from at least one residue selected from the group consisting of G34, P45, G55, N69, T83, A99, and A109 to the C-terminus residue of SEQ ID NO: 1.

6. The composition according to claim 1, wherein the DNA binding protein is zinc-finger protein (ZF protein) or transcription activator-like effector (TALE) array.

7. The composition according to claim 1, wherein the DNA binding protein is linked via peptide linker comprising 2 to 40 amino acid residues.

8. The composition according to claim 7, wherein the linker comprises:
2a.a linker: GS;
5a.a linker: TGEKQ;
10a.a linker: SGAQGSTLDF;
16a.a linker: SGSETPGTSESATPES;
24 aa linker: SGTPHEVGVYTLSGTPHEVGVYTL; or
32a.a linker: GSGGSSGGSSGSETPGTSESATPESSGGSSGGS.

9. The composition according to claim 1, wherein the DNA binding protein is zinc-finger protein, and each of the first and second splits is independently linked to the N-terminus or C-terminus of the zinc-finger protein.

10. The composition according to claim 1, wherein the DNA binding protein is TALE array, and wherein a single TALE array is linked to one terminus of the split, or first and second TALE arrays are linked to the first and second splits.

11. The composition according to claim 1, wherein the adenine deaminase is linked to the N-terminus or C-terminus of the DNA-binding protein or cytosine deaminase.

12. The composition according to claim 1, wherein the adenine deaminase is deoxyadenine deaminase derived from TadA.

13. The composition according to claim 1, comprising chloroplast transit peptide or nucleic acid encoding the chloroplast transit peptide.

14. The composition according to claim 1, comprising mitochondrial targeting signal (MTS) or nucleic acid encoding the mitochondrial targeting signal.

15. The composition according to claim 1, which does not comprise uracil DNA-glycosylase inhibitor (UGI).

16. The composition according to claim 1, wherein the composition is delivered into plant cells by means of:
bombardment using a gene gun;
protoplast transfection mediated by polyethylene glycol (PEG);
protoplast transfection via electroporation; or
protoplast injection via microinjection.

17. The composition according to claim 1, wherein the nucleic acid is delivered into plant cells by means of:
transformation using *Agrobacterium tumefaciens or Agrobacterium rhizogene;*
viral transfection;
bombardment using a gene gun;
protoplast transfection mediated by polyethylene glycol (PEG);
protoplast transfection via electroporation; or
protoplast injection via microinjection.

18. A method for editing organellar DNA in plant cells, comprising a step of treating plant cells with the composition according to any one of claims 1 to 17.
